(19) Europäisches Patentamt
European Patent Office
Office européen des brevets

(11) Numéro de publication: **0 266 283 B1**

# (12) FASCICULE DE BREVET EUROPEEN

(45) Date de publication de fascicule du brevet: **22.04.92**

(21) Numéro de dépôt: **87420273.2**

(22) Date de dépôt: **09.10.87**

(51) Int. Cl.⁵: **C07C 49/403**, C07C 35/08, C07C 409/14, C07C 45/33, C07C 45/53, C07C 29/50, C07C 29/132

## (54) Procédé de préparation d'un mélange renfermant du cyclohéxanol et de la cyclohéxanone à partir du cyclohéxane.

(30) Priorité: **10.10.86 FR 8614282**

(43) Date de publication de la demande:
**04.05.88 Bulletin 88/18**

(45) Mention de la délivrance du brevet:
**22.04.92 Bulletin 92/17**

(84) Etats contractants désignés:
**BE DE ES FR GB IT NL**

(56) Documents cités:
**EP-A- 0 027 937**
**GB-A- 716 820**
**GB-A- 1 212 824**
**US-A- 2 684 984**
**US-A- 4 341 907**

**CHEMICAL ABSTRACTS, vol. 95, no. 3, juillet 1981, page 633, résumé no. 24369q, Columbus, Ohio, US; & SU-A-806 670**

(73) Titulaire: **RHONE-POULENC CHIMIE**
**25, quai Paul Doumer**
**F-92408 Courbevoie Cédex(FR)**

(72) Inventeur: **Costantini, Michel**
**10, rue du Docteur Bonhomme**
**F-69003 Lyon(FR)**
Inventeur: **Igersheim, Françoise**
**21, rue Florian**
**F-69100 Villeurbanne(FR)**

(74) Mandataire: **Varnière-Grange, Monique et al**
**RHONE-POULENC INTERSERVICES Service Brevets Chimie Centre de Recherches des Carrières B.P. 62**
**F-69192 Saint-Fons Cédex(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services

CHEMICAL ABSTRACTS, vol. 84, 1976, page 502, colonne de droite, résumé no 89889b, Columbus, Ohio, US; IL-A-41 114

BULLETIN OF THE ACADEMY OF SCIENCE OF THE USSR, vol. 29, no. 7, pt. I, juillet 1980, pages 1081-1086, Plenum Publishing Corporation, New York, US; L.V. PETROV et al.: "Decomposition of tert-butyl hydroperoxide in acetonitrile catalyzed by antimony pentachloride"

## Description

La présente invention est relative à un procédé de préparation d'un mélange renfermant du cyclohexanol et de la cyclohexanone à partir du cyclohexane.

Elle concerne plus particulièrement un procédé dans lequel le cyclohexane est oxydé en présence d'air pour former un mélange renfermant de l'hydroperoxyde de cyclohexyle désigné dans ce qui suit par HPOCH, cet hydroperoxyde étant alors décomposé. Les produits principalement formés lors de la décomposition sont le cyclohexanol (OL) et la cyclohexanone (ONE). Ces produits sont convertis par oxydation en acide adipique, une des matières premières des polyamides. La cyclohexanone est aussi un intermédiaire du caprolactame, produit qui entre dans la fabrication de certains polyamides.

D. Mansuy et ses collaborateur dans Angew. Chem. Int. Ed. Engl 19, 1980 N°11 pages 909-910, on décrit l'oxydation du cyclohexane par l'hydroperoxyde de cumyle en présence du complexe :

$Mn^{III}(TPP)Cl$,

TPP désignant la tétraphénylporphyrine.

Toutefois les rendements restent faibles. Le catalyseur est coûteux et sensible à l'oxygène ; il est en outre nécessaire de travailler en présence d'un solvant.

Si le brevet français n° 1 263 449 décrit la décomposition de l'hydroperoxyde de cyclohexyle au sein d'un mélange résultant de l'oxydation du cyclohexane, avant séparation de ce dernier, en présence d'une combinaison manganèse et de cuivre pouvant être mise en dispersion dans le mélange réactionnel, il s'agit là d'une décomposition totale sans pour autant oxyder le cyclohexane.

Par ailleurs, dans la demande de brevet européen EP-A-0 027 937 est décrit un procédé amélioré d'oxydation du cyclohexane pour former un mélange réactionnel renfermant de l'HPOCH et de décomposition de cet hydroperoxyde en présence de cyclohexane (matière de départ) pour former un mélange contenant du cyclohexanol et de la cyclohexanone dans lequel le perfectionnement comprend l'utilisation comme catalyseur dans le stade d'oxydation et/ou de décomposition, d'un complexe d'un métal de transition et de composés de la classe des bis(pyridylimino)-1,3 isoindolines.

Un aspect important de ce procédé réside dans la participation du cyclohexane qui contribue à une conversion supérieure dudit cyclohexane, en produits d'oxydation "utiles", à celle qui serait normalement observée lors de la seule décomposition de l'HPOCH.

Toutefois ce procédé présente deux inconvénients majeurs, à savoir, d'une part le recours à des coordinats très coûteux et/ou difficiles à préparer et, d'autre part la sensibilité desdits coordinats aux oxydants.

Il a maintenant été trouvé un procédé permettant de pallier les insuffisances des procédés antérieurs.

La présente invention a donc pour objet un procédé de production d'un mélange de cyclohexanol et de cyclohexanone dans lequel on oxyde le cyclohexane par l'air pour produire un mélange contenant de l'hydroperoxyde de cyclohexyle et dans lequel l'hydroperoxyde de cyclohexyle est décomposé en présence de cyclohexane pour former le cyclohexanol et la cyclohexanone,

caractérisé en ce que

la décomposition de l'hydroperoxyde de cyclohexyle est réalisée par la mise en contact en phase liquide, à une température comprise entre 60 et 160°C d'un mélange réactionnel contenant du cyclohexane et de l'ordre de 0,1 à 10 % en poids d'hydroperoxyde de cyclohexyle en présence d'une quantité catalytiquement efficace de manganèse ou d'un composé du maganèse, d'un acide de Bronsted dont le $pk_a$ est supérieur ou égal à 0,7 et d'un coordinat pyridinique répondant à l'une quelconque des formules (I) à (III) ci-après, et le cas échéant en présence d'oxygène moléculaire :

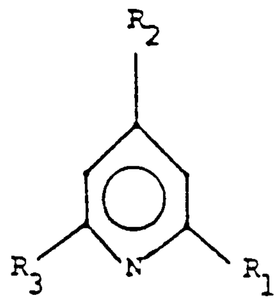

(I)

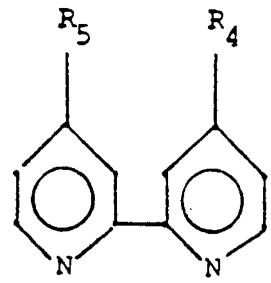

(II)

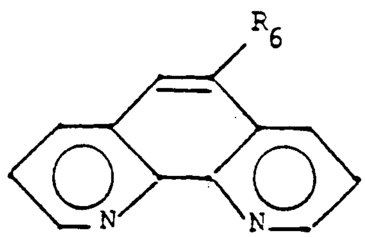

(III)

. $R_1$, $R_2$ et $R_3$, identiques ou différents, représentent un atome d'hydrogène, un radical alkyle comportant au maximum 4 atomes de carbone, linéaire ou ramifié mais non substitué par des groupements susceptibles d'interférer avec la réaction principale, ou un groupe carboxyle,

. $R_4$ et $R_5$, identiques ou différents, ont la signification donnée ci-avant pour $R_1$, $R_2$ et $R_3$, sous réserve toutefois que lorsque $R_4$ ou $R_5$ représente un groupe carboxyle, l'autre symbole représente l'hydrogène ou un groupe carboxyle et

. $R_6$ représente un atome d'hydrogène, un radical alkyle comportant au maximum 4 atomes de carbone, linéaire ou ramifié mais non substitué par des groupements susceptibles d'interférer avec la réaction principale, ou un groupe nitro.

Ainsi la mise en oeuvre du procédé selon l'invention nécessite la présence d'une quantité catalytiquement efficace de manganèse ou d'un composé du manganèse.

Par quantité catalytiquement efficace on entend une teneur suffisante pour donner une vitesse de réaction industriellement acceptable, vitesse qui dépend également des autres conditions de la réaction, tout en n'alourdissant pas les opérations de recyclage et/ou de purification des produits visés.

En général la teneur en manganèse du milieu réactionnel liquide est d'au moins 1 millimole par litre (mmol/l). On n'observe pas d'avantage à dépasser une concentration de l'ordre 25 mmol/l.

Le manganèse métallique finement divisé et plus généralement n'importe quel composé du managnèse sont susceptibles de convenir à la mise en oeuvre du présent procédé. A cet effet les composés du manganèse (II) comme ceux du manganèse (III) peuvent être employés. A titre d'exemple de composés utilisables dans le cadre du présent procédé on peut citer :
l'acétate manganeux, l'acétate manganique et plus généralement les carboxylates de manganèse (II) ou (III), le sulfate, le nitrate et le carbonate manganeux et l'acétylacétonate de manganèse (III).

On préfère recourir aux acétates de manganèse ou à l'acétylacétonate de manganèse (III).

La mise en oeuvre du présent procédé requiert la présence d'un coordinat pyridinique répondant à l'une quelconque des formules (I) à (III) ci-avant.

S'agissant des coordinats de formule (I), on envisage plus particulièrement de recourir à ceux pour lesquels les symboles $R_1$ à $R_3$ représentent indépendamment l'hydrogène, un radical méthyl ou un groupe carboxyle. A titre d'exemples de tels coordinats on peut citer : la pyridine, la picoline-2, la picoline-4, la lutidine-2,6, la collidine-2,4,6 (ou triméthyl-2,4,6 pyridine), la carboxy-2 pyridine et la carboxy-4 pyridine.

S'agissant des coordinats de formule (II), on recourt avantageusement à ceux pour lesquels les symboles $R_4$ et $R_5$ de la formule en cause représentent indépendamment un atome d'hydrogène ou un radical méthyle, ou lorsque $R_4$ (ou $R_5$) représente un groupe carboxyle, $R_5$ (ou $R_4$) représente un atome d'hydrogène ou un groupe carboxyle.

A titre d'exemples de tels coordinats on peut citer : la bipyridine-2,2' (ou bipyridyle-2,2'), la diméthyl-4,4' bipyridine-2,2' et la dicarboxy-4,4' bipyridine-2,2'.

Enfin, les coordinats de formule (III) plus particulièrement envisagés correspondent à ceux pour lesquels le symbole $R_6$ représente un atome d'hydrogène ou un radical méthyle et sont respectivement appelés la phénantroline-1,10 et la méthyl-5 phénantroline-1,10.

La bipyridine-2,2' et la dicarboxy-4,4' bipyridine-2,2' sont particulièrement préconisées.

En général, la quantité de coordinat pyridinique est telle que le rapport atomique N/Mn soit compris entre 1 et 10 et, de préférence entre 3 et 5. Pour une bonne mise en oeuvre de l'invention ce rapport sera de l'ordre de 4.

Comme indiqué en tête du présent mémoire, le présent procédé nécéssite la présence d'un acide de Bronsted dont le $pK_a$ déterminé à 25 ° C est supérieur à 0,7. Bien entendu tout ou partie de cet acide peut provenir du coordinat pyridinique porteur d'un ou deux groupements carboxyles.

Parmi les acides utilisables dans le cadre du présent procédé on peut citer l'acide benzoïque, les acides monocarboxyliques aliphatiques saturés renfermant de 1 à 12 atomes de carbone dans la chaîne principale et pouvant comporter au maximum trois substituants choisis parmi les radicaux alkyles en $C_1$-$C_4$, les atomes de chlore et de brome.

A titre d'exemples d'acides ne provenant pas des coordinats pyridiniques convenant à la mise en oeuvre du procédé selon l'invention, on peut citer l'acide acétique, l'acide benzoïque, l'acide monochloracétique, l'acide hexanoïque et l'acide dichloroacétique.

Il est à noter que l'acide hexanoïque ou caproïque qui est un coproduit de l'oxydation du cyclohexane convient plus particulièrement bien.

En général la quantité d'acide mise en oeuvre est telle que le rapport atomique $H+$/Mn soit compris entre 2 et 20 et, de préférence entre 8 et 15.

On opère donc en deux opérations distinctes, l'oxydation est alors conduite en l'absence de catalyseur ou en présence d'un système catalytique différent comme par exemple celui décrit dans le brevet des Etats Unis d'Amérique n° 3 923 895.

En effet, la participation du cyclohexane dans le stade de décomposition de l'hydroperoxyde de cyclohexyle au sens de la présente invention, peut être accrue lorsque cette décomposition est l'objet d'une opération distincte.

S'agissant de l'oxydation les conditions seront avantageusement les suivantes :

La température sera comprise entre 80 et 160 ° C inclus et la pression nécessaire au maintien du cyclohexane dans la phase liquide variera selon la température choisie entre la pression atmosphérique et 20 bars.

La réaction de décomposition est conduite en phase liquide, la concentration de l'hydroperoxyde de cyclohexyle étant comprise entre 0,1 et 10 % en poids. Cette concentration est avantageusement comprise entre 0,5 et 8 %.

On peut faire appel à divers solvants tels les alcanes parmi lesquels on citera plus particulièrement l'hexane. l'heptane et l'isooctane ; les cycloalcanes parmi lesquels on mentionnera à titre illustratif le cyclohexane et le cyclooctane, les hydrocarbures aromatiques tel le benzène, le toluène et le xylène et leurs mélanges.

Toutefois il est à noter que l'hydroperoxyde de cyclohexyle étant produit sous la forme d'une solution dans le cyclohexane par oxydation comme cela a été indiqué ci-avant, la réaction de décomposition est avantageusement réalisée sur une solution provenant de l'oxydation du cyclohexane dans laquelle la concentration en hydroperoxyde de cyclohexyle est comprise dans les limites indiquées précédemment. Cette solution peut être utilisée en l'état ou après élimination de certains constituants de manière en soi connue. Il est également possible d'utiliser une solution d'hydroperoxyde de cyclohexyle dans le cyclohexane sensiblement pur.

Si la décomposition de l'hydroperoxyde de cyclohexyle peut être réalisée en l'absence d'oxygène ou d'air, la présence d'oxygène ou d'air est avantageuse dans la mesure où elle augmente le rendement en cyclohexanol-cyclohexanone sans pour autant exercer d'effet néfaste sur la décomposition de l'hydroperoxyde.

Il conviendra néanmoins pour des raisons de sécurité de s'assurer que la quantité d'oxygène dans la phase gazeuse ou ciel du réacteur reste inférieure à 5 % en volume.

La température est généralement comprise entre 60 et 160 ° C. Au delà de 120 ° C, intervient une décomposition par voie thermique qui peut être non souhaitable.

La pression atmosphérique ou supérieure à la pression atmosphérique sera suffisante pour maintenir le cyclohexane en phase liquide.

La durée de réaction (ou le temps de séjour) est généralement compris entre 10 minutes et 5 heures et peut être ajustée compte tenu des objectifs de production, de la quantité des divers constituants du

système catalytique engagée et des autres paramètres de la réaction.

En fin de réaction les produits peuvent être récupérés par tout moyen approprié, par exemple par distillation.

Les exemples ci-après illustrent la présente invention.

Dans ces exemples les conventions suivantes sont utilisées :

- TT (HPOCH) désigne le taux de transformation de l'hydroperoxyde de cyclohexyle engagé
- RT (OLONE) désigne le rendement apparent en cyclohexanone et cyclohexanol c'est à dire le rapport du nombre de moles de cyclohexanone et de cyclohexanol formées au nombre de moles d'hydroperoxyde de cyclohexyle transformées.

un rendement supérieur à 100 % indique qu'une fraction des produits en cause provient de l'oxydation du cyclohexane.

EXEMPLE 1 :

Dans un réacteur muni d'un agitateur central et d'un réfrigérant, on charge sous atmosphère inerte :

- 84 g (1 mole) de cyclohexane,
- 90 mg (0,25 mmol) d'acétylacétonate de manganèse [$Mn(acac)_3$],
- 118 mg (0,75 mmol ) de bipyridine-2,2' et,
- 178 mg (1,53 mmol) d'acide caproïque ($pK_a$ = 4,4).

Le mélange est chauffé à 60 °C et lorsque cette température est atteinte on ajoute le plus rapidement possible :

- 6 g d'hydroperoxyde de cyclohexyle à 97 %.

L'agitation est maintenue jusqu'à ce que la teneur en oxygène peroxydique soit nulle ou stable (soit environ 6 heures dans le cas présent). Cette durée est appelée temps de réaction dans ce qui suit. Le mélange est refroidi et l'oxygène peroxydique résiduel est réduit par la triphénylphosphine. Le mélange est ensuite analysé par chromatographie en phase vapeur.

Il contient :

- Hydroperoxyde résiduel : 432,7 mg soit 3,7 mmol
  Cyclohexanol : 3 680 mg soit 36,8 mmol
  Cyclohexanone : 2 018 mg soit 20,6 mmol

Ce qui correspond à :

TT (HPOCH) 92,5 %
RT (OLONE) 115,8 %

ESSAI TEMOIN (a) :

On reproduit l'exemple I ci-dessus en omettant de charger l'acide caproïque.

Le temps de réaction est de 5 heures et les résultats sont les suivants :

TT (HPOCH) 56 %
RT (OLONE) 106 %

ESSAI TEMOIN (b) :

On reproduit l'exemple I ci-dessus en omettant de charger la bipyridine.

Le temps de réaction est de 5 heures et les résultats sont les suivants :

TT (HPOCH) 65 %
RT (OLONE) 86 %

Les conditions particulières ainsi que les résultats obtenus dans l'exemple 1 et les essais temoins (a) et (b) sont consignés dans le tableau I ci-après.

Tableau I

| Réf. | Acide présent | Bipyridine présente | temps en heures | TT (%) (HPOCH) | RT (%) (OLONE) |
|---|---|---|---|---|---|
| Ex 1 | oui | oui | 6 | 92,5 | 115,8 |
| a | non | oui | 5 | 56 | 106,0 |
| b | oui | non | 5 | 65 | 86,0 |

Les essais temoins (a) et (b) montrent qu'en l'absence d'acide ou de bipyridine le taux de transformation reste faible et la sélectivité en OLONE est insuffisante.

EXEMPLE 2 :

On reproduit l'exemple I mais en remplaçant la bipyridine par :

- 138 mg (soit 0,75 mmol) de diméthyl-4,4'bipyridine-2,2'.

Les résultats obtenus en 4 heures de réaction sont les suivants :

TT (HPOCH) 98,4 %
RT (OLONE) 116,5 %

EXEMPLE 3 :

On reproduit l'exemple I mais en remplaçant la bipyridine et l'acide caproïque par :

- 183 mg (soit 0,75 mmol) de dicarboxy-4,4'bipyridine-2,2'.

Les résultats obtenus en 5 heures de réaction sont les suivants :

TT (HPOCH) 77,5 %
RT (OLONE) 115,5 %

EXEMPLE 4 :

On reproduit l'exemple I mais en chargeant :

- 300 mg (soit 2,5 mmol) d'acide caproïque

Les résultats obtenus en 3h 30 de réaction sont les suivants :

TT (HPOCH) 94 %
RT (OLONE) 120 %

EXEMPLE 5 :

On reproduit l'exemple I mais en chargeant une quantité équivalente de manganèse sous forme de $Mn(NO_3)_2$

Les résultats obtenus au bout de 5 heure de réactions sont les suivants :

TT (HPOCH) 100 %
RT (OLONE) 110 %

EXEMPLES 6 à 8 :

Une série d'essais est réalisée avec l'appareillage et selon le mode opératoire de l'exemple 1, la charge renfermant pour chaque essai :

- 84 g (1 mole) de cyclohexane,
- 90 mg (0,25 mmol) d'acétylacétonate de manganèse [$Mn(acac)_3$],
- 140 mg (0,75 mmol) de diméthyl-4,4'bipyridine-2,2' et ,
- 1,25 mmol d'acide dont la nature est précisée pour chaque essai.

La température est de 60 °C.

Les conditions particulières ainsi que les résultats obtenus pour chaque essai sont rassemblés dans le tableau II ci-après.

Tableau II

| EX. | Acide | | temps en heures | TT (%) (HPOCH) | RT (%) (OLONE) |
|---|---|---|---|---|---|
| | nature | $pK_a$ | | | |
| 6 | $CH_2ClCOOH$ | 2,85 | 3,5 | 100 | 112,5 |
| 7 | $CHCl_2COOH$ | 1,5 | 1 | 100 | 107 |
| 8 | $CCl_3COOH$ | 0,7 | 6 | 89 | 118 |

EXEMPLE 9 :

Dans un appareil analogue à celui décrit pour l'exemple 1, on charge :

- 84g (1 mole) de cyclohexane,
- 90 mg (0.25 mmol) d'acétylacétonate de manganèse [Mn(acac) ],$_3$
- 137 mg (0,75 mmol) de diméthyl-4,4'bipyridine-2,2' et ,
- 178 mg (1.5 mmol) d'acide caproïque.

Le mélange est alors porté à 80 °C sous azote et on lui ajoute 6g d'HPOCH. La solution est alors placée sous un débit de 10 l/h (mesuré dans les conditions normales de pression et de température) d'air saturé en cyclohexane.

Les résultats obtenus en 5 heures de réaction sont les suivants :

TT (HPOCH) 93 %
RT (OLONE) 135 %

**Revendications**

**1.** Procédé de production d'un mélange de cyclohexanol et de cyclohexanone dans lequel on oxyde le cyclohexane par l'air pour produire un mélange contenant de l'hydroperoxyde de cyclohexyle et dans lequel l'hydroperoxyde de cyclohexyle est décomposé en présence de cyclohexane pour former le cyclohexanol et la cyclohexanone caractérisé en ce que la décomposition de l'hydroperoxyde de cyclohexyle est réalisée par la mise en contact en phase liquide, à une température comprise entre 60 et 160 °C d'un mélange réactionnel contenant du cyclohexane et de l'ordre de 0,1 à 10 % en poids d'hydroperoxyde de cyclohexyle en présence d'une quantité catalytiquement efficace de manganèse ou d'un composé du manganèse, d'un acide de Bronsted dont le $pk_a$ déterminé à 25 °C est supérieur ou égal à 0,7 et d'un coordinat pyridinique répondant à l'une quelconque des formules (I) à (III) ci-après, et le cas échéant en présence d'oxygène moléculaire :

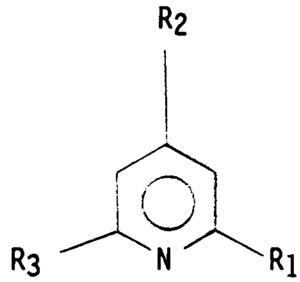

(I)

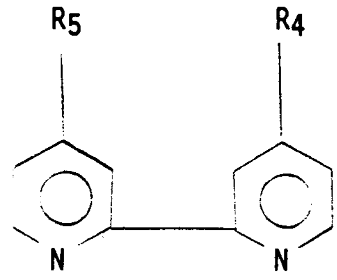

(II)

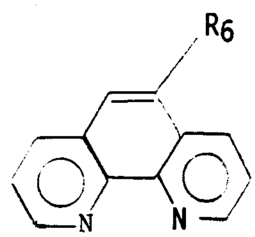

(III)

. $R_1$, $R_2$, et $R_3$, identiques ou différents, représentent un atome d'hydrogène, un radical alkyle comportant au maximum 4 atomes de carbone, linéaire ou ramifié mais non substitué par des groupements susceptibles d'interférer avec la réaction principale, ou un groupe carboxyle.

. $R_4$ et $R_5$, identiques ou différents, ont la signification donnée ci-avant pour $R_1$, $R_2$ et $R_3$, sous réserve toutefois que lorsque $R_4$ ou $R_5$ représente un groupe carboxyle, l'autre symbole représente l'hydrogène ou un groupe carboxyle, et

. $R_6$ représente un atome d'hydrogène, un radical alkyle comportant au maximum 4 atomes de carbone, linéaire ou ramifié mais non substitué par des groupements susceptibles d'interférer avec la réaction principale, ou un groupe nitro.

2. Procédé selon la revendication 1, caractérisé en ce que la décomposition est réalisée en présence d'oxygène moléculaire.

3. Procédé selon la revendication 1 ou 2, caractérisé en ce que la concentration du manganèse dans le milieu réactionnel liquide est comprise entre 1 et 25 mmol/l.

4. Procédé selon l'une quelconque des revendications précédentes, caractérisé en ce que la quantité de coordinat pyridinique est telle que le rapport atomique N/Mn soit compris entre 1 et 10.

5. Procédé selon l'une quelconque des revendications précédentes, caractérisé en ce que la quantité de l'acide de Bronsted est telle que le rapport atomique $H+/Mn$ soit compris entre 2 et 20.

6. Procédé selon l'une quelconque des revendications précédentes, caractérisé en ce que le coordinat pyridinique répond à l'une quelconque des formules (I) à (II) figurant dans la revendication 1 dans lesquelles :

. $R_1$, $R_2$ et $R_3$, identiques ou différents, représentent indépendamment l'hydrogène, un radical méthyle ou un groupe carboxyle,

. $R_4$ et $R_5$, représentent indépendamment un atome d'hydrogène ou un radical méthyle ou lorsque $R_4$ (ou $R_5$) représente un groupe carboxyle, $R_5$ (ou $R_4$) représente un atome d'hydrogène ou un groupe carboxyle.

. $R_6$ représente un atome d'hydrogène ou un radical méthyle.

7. Procédé selon l'une quelconque des revendications précédentes, caractérisé en ce que le coordinat pyridinique répond à la formule (II) :

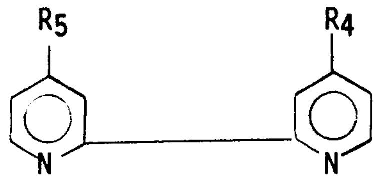

(II)

dans laquelle :

. $R_4$ et $R_5$, représentent indépendamment un atome d'hydrogène ou un radical méthyle ou lorsque $R_4$ (ou $R_5$) représente un groupe carboxyle, $R_5$ (ou $R_4$) représente un atome d'hydrogène ou un groupe carboxyle.

**8.** Procédé selon la revendication 7, caractérisé en ce que le coordinat pyridinique est choisi parmi la bipyridine-2,2' et la dicarboxy-4,4' bipyridine-2,2'.

**9.** Procédé selon l'une quelconque des revendications précédentes, caractérisé en ce que l'acide de Bronsted est choisi parmi les coordinats pyridiniques répondant à l'une quelconque des formules (I) à (III) figurant dans la revendication 1, et portant un ou deux groupes carboxyles.

**10.** Procédé selon l'une quelconque des revendications précédentes, caractérisé en ce que l'acide de Bronsted est choisi parmi l'acide benzoïque et les acides monocarboxyliques aliphatiques saturés renfermant de 1 à 12 atomes de carbone dans la chaîne principale et pouvant comporter au maximum trois substituants choisis parmi les radicaux alkyles en $C_1$-$C_4$, les atomes de chlore, de brome.

**11.** Procédé selon la revendication 10, caractérisé en que l'acide de Bronsted est l'acide caproïque.

**12.** Procédé selon l'une quelconque des revendications précédentes, caractérisé en ce que le manganèse est introduit dans le milieu réactionnel sous forme d'un composé du manganèse (II) ou du manganèse (III).

**13.** Procédé selon la revendication 12, caractérisé en ce que le manganèse est introduit dans le milieu réactionnel sous forme d'un acétate.

**14.** Procédé selon la revendication 12, caractérisé en ce que le manganèse est introduit dans le milieu réactionnel sous forme de l'acétylacétonate de manganèse (III).

**15.** Procédé selon l'une quelconque des revendications précédentes, caractérisé en ce que la température de décomposition est inférieure à 120 ° C.

**16.** Procédé selon l'une quelconque des revendications précédentes, caractérisé en ce que la concentration initiale de l'hydroperoxyde de cyclohexyle est comprise entre 0,5 et 8 % en poids.

## Claims

**1.** Process for the production of a mixture of cyclohexanol and cyclohexanone, in which cyclohexane is oxidized by air to produce a mixture containing cyclohexyl hydroperoxide and in which the cyclohexyl hydroperoxide is decomposed in the presence of cyclohexane to form cyclohexanol and cyclohexanone, characterised in that the decomposition of cyclohexyl hydroperoxide is carried out by bringing a reaction mixture containing cyclohexane and approximately 0.1 to 10% by weight of cyclohexyl hydroperoxide into contact, in the presence of a catalytically effective quantity of manganese or a manganese compound, a Brönsted acid having a $pK_a$ determined at 25 ° C of greater than or equal to 0.7, at a temperature between 60 and 160 ° C, and where appropriate in the presence of molecular oxygen, with a pyridine ligand corresponding to any one of the formulae (I) to (III) below:

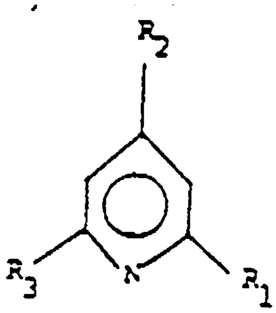

(I)

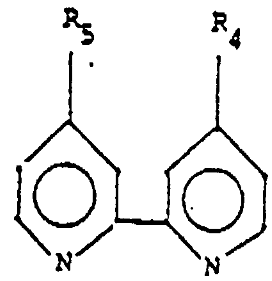

(II)

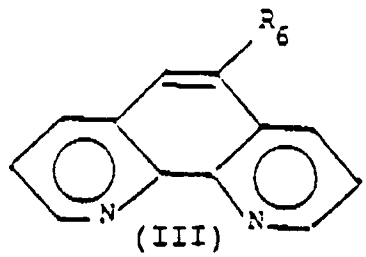

(III)

;

$R_1$, $R_2$ and $R_3$, which may be identical or different, represent a hydrogen atom, a straight-chain or branched alkyl radical containing at most 4 carbon atoms, which is not substituted, however, by groups which are able to interfere with the main reaction, or a carboxyl group,

$R_4$ and $R_5$, which may be identical or different, have the meaning given above for $R_1$, $R_2$ and $R_3$, on condition, however, that when $R_4$ or $R_5$ represents a carboxyl group, the other symbol represents hydrogen or a carboxyl group, and

$R_6$ represents a hydrogen atom, a straight-chain or branched alkyl radical containing at most 4 carbon atoms, which is not substituted, however, by groups which are able to interfere with the main reaction, or a nitro group.

**2.** Process according to Claim 1, characterised in that the decomposition is carried out in the presence of molecular oxygen.

**3.** Process according to Claim 1 or 2, characterised in that the manganese concentration in the liquid reaction medium is between 1 and 25 mmol/l.

**4.** Process according to any one of the preceding claims, characterised in that the quantity of pyridine ligand is such that the atomic ratio N:Mn is between 1 and 10.

**5.** Process according to any one of the preceding claims, characterised in that the quantity of Brönsted acid is such that the atomic ratio $H^+$:Mn is between 2 and 20.

**6.** Process according to any one of the preceding claims, characterised in that the pyridine ligand corresponds to any one of the formulae (I) to (II) appearing in Claim 1 in which:

$R_1$, $R_2$ and $R_3$, which may be identical or different, independently represent hydrogen, a methyl radical or a carboxyl group,

$R_4$ and $R_5$ independently represent a hydrogen atom or a methyl radical or, when $R_4$ (or $R_5$) represents a carboxyl group, $R_5$ (or $R_4$) represents a hydrogen atom or a carboxyl group, and

$R_6$ represents a hydrogen atom or a methyl radical.

**7.** Process according to any one of the preceding claims, characterised in that the pyridine ligand corresponds to the formula (II):

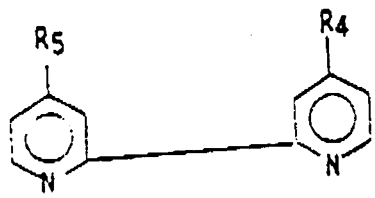

(II)

in which:
$R_4$ and $R_5$ independently represent a hydrogen atom or a methyl radical or, when $R_4$ (or $R_5$) represents a carboxyl group, $R_5$ (or $R_4$) represents a hydrogen atom or a carboxyl group.

**8.** Process according to claim 8, characterised in that the pyridine ligand is chosen from amongst 2,2'-bipyridine and 4,4'-dicarboxy-2,2'-bipyridine.

**9.** Process according to any one of the preceding claims, characterised in that the Brönsted acid is chosen from amongst pyridine ligands corresponding to any one of the formulae (I) to (III) appearing in Claim 1 and carrying one or two carboxyl groups.

**10.** Process according to any one of the preceding claims, characterised in that the Brönsted acid is chosen from amongst benzoic acid and saturated aliphatic monocarboxylic acids containing from 1 to 12 carbon atoms in the main chain and which may contain at most three substituents chosen from amongst $C_1$-$C_4$ alkyl radicals and chlorine and bromine atoms.

**11.** Process according to Claim 10, characterised in that the Brönsted acid is caproic acid.

**12.** Process according to any one of the preceding claims, characterised in that the manganese is introduced into the reaction medium in the form of a manganese(II) or manganese(III) compound.

**13.** Process according to Claim 12, characterised in that the manganese is introduced into the reaction medium in the form of an acetate.

**14.** Process according to Claim 13, characterised in that the manganese is introduced into the reaction medium in the form of manganese(III) acetylacetonate.

**15.** Process according to any one of the preceding claims, characterised in that the decomposition temperature is below 120 °C.

**16.** Process according to any one of the preceding claims, characterised in that the initial cyclohexyl hydroperoxide concentration is between 0.5 and 8% by weight.

**Patentansprüche**

**1.** Verfahren zur Herstellung eines Gemisches aus Cyclohexanol und Cyclohexanon, bei dem man Cyclohexan mit Luft oxidiert, um ein Gemisch, das Cyclohexylhydroperoxid enthält, zu erzeugen, und bei dem das Cyclohexylhydroperoxid in Gegenwart von Cyclohexan zersetzt wird, um Cyclohexanol und Cyclohexanon zu bilden, dadurch gekennzeichnet, daß die Zersetzung des Cyclohexylhydroperoxids bewirkt wird, indem in flüssiger Phase bei einer Temperatur im Bereich von 60 bis 160 °C ein Reaktionsgemisch, das Cyclohexan und 0,1 bis 10 Gew.-% Cyclohexylhydroperoxid enthält, in Gegenwart einer katalytisch wirksamen Menge Mangan oder einer Manganverbindung mit einer Brönsted-Säure, deren bei 25 °C bestimmter pKa ≧ 0,7 ist und mit einer Pyridin-Koordinationsverbindung, die einer der nachfolgenden Formeln (I) bis (III) entspricht, und gegebenenfalls in Gegenwart von molekularem Sauerstoff, zusammenbringt:

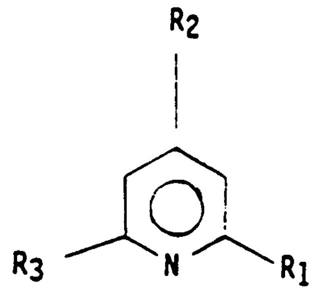

(I)

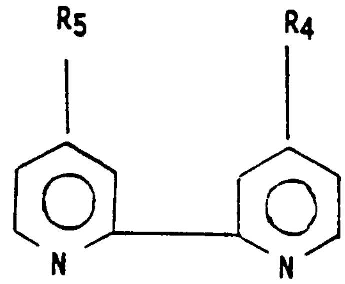

(II)

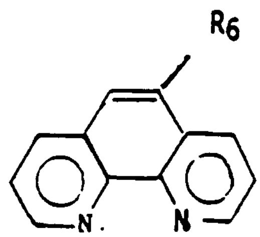

(III)

worin

. $R_1$, $R_2$ und $R_3$ gleich oder verschieden sind und (jeweils) ein Wasserstoffatom, eine Alkylgruppe mit maximal 4 Kohlenstoffatomen, linear oder verzweigt, aber nicht substituiert mit Gruppen, die die Hauptreaktion stören können, oder eine Carboxylgruppe bedeuten,

. $R_4$ und $R_5$ gleich oder verschieden sind und die vorstehend für $R_1$, $R_2$ und $R_3$ angegebene Bedeutung haben, mit der Maßgabe, daß, wenn $R_4$ oder $R_5$ eine Carboxylgruppe bedeutet, der andere Substituent Wasserstoff oder eine Carboxylgruppe ist, und

- $R_6$ ein Wasserstoffatom, eine Alkylgruppe mit maximal 4 Kohlenstoffatomen, linear oder verzweigt, aber nicht substituiert mit Gruppen, die die Hauptreaktion stören können, oder eine Nitrogruppe bedeutet.

**2.** Verfahren nach Anspruch 1, dadurch gekennzeichnet, daß die Zersetzung in Gegenwart von molekularem Sauerstoff durchgeführt wird.

**3.** Verfahren nach Anspruch 1 oder 2, dadurch gekennzeichnet, daß die Konzentration von Mangan im flüssigen Reaktionsmedium 1 bis 25 mmol/l beträgt.

**4.** Verfahren nach einem der vorangehenden Ansprüche, dadurch gekennzeichnet, daß die Pyridin-Koordinationsverbindung in einer solchen Menge vorhanden ist, daß das Atomverhältnis N/Mn 1 bis 10 beträgt.

**5.** Verfahren nach einem der vorangehenden Ansprüche, dadurch gekennzeichnet, daß die Brönsted-Säure in solcher Menge vorhanden ist, daß das Atomverhältnis $H^+/Mn$ 2 bis 20 beträgt.

**6.** Verfahren nach einem der vorangehenden Ansprüche, dadurch gekennzeichnet, daß die Pyridin-Koordinationsverbindung einer der im Anspruch 1 angegebenen Formeln (I) bis (II) entspricht, in denen:

. $R_1$, $R_2$ und $R_3$ gleich oder verschieden sind und jeweils für Wasserstoff, eine Methyl- oder eine Carboxylgruppe stehen,

. $R_4$ und $R_5$ jeweils ein Wasserstoffatom oder eine Methylgruppe bedeuten oder, wenn $R_4$ (oder $R_5$) für eine Carboxylgruppe steht, $R_5$ (oder $R_4$) ein Wasserstoffatom oder eine Carboxylgruppe bedeutet,

. $R_6$ ein Wasserstoffatom oder eine Methylgruppe ist.

**7.** Verfahren nach einem der vorangehenden Ansprüche, dadurch gekennzeichnet, daß die Pyridin-Koordinationsverbindung der Formel (II):

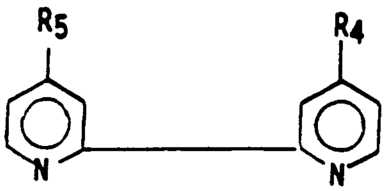

(II)

entspricht, in der

. $R_4$ und $R_5$ unabhängig voneinander ein Wasserstoffatom oder eine Methylgruppe bedeuten oder, wenn $R_4$ (oder $R_5$) eine Carboxylgruppe bedeutet, $R_5$ (oder $R_4$) für ein Wasserstoffatom oder eine Carboxylgruppe steht.

**8.** Verfahren nach Anspruch 7, dadurch gekennzeichnet, daß die Pyridin-Koordinationsverbindung aus 2,2'-Bipyridin und 4,4'-Dicarboxy-2,2'-bipyridin ausgewählt wird.

**9.** Verfahren nach einem der vorangehenden Ansprüche, dadurch gekennzeichnet, daß die Brönsted-Säure aus den Pyridin-Koordinationsverbindungen ausgewählt wird, die irgendeiner der Formeln (I) bis (III) im Anspruch 1 entsprechen, und die ein oder zwei Carboxylgruppen tragen.

**10.** Verfahren nach einem der vorangehenden Ansprüche, dadurch gekennzeichnet, daß die Brönsted-Säure aus Benzoesäure und den aliphatischen gesättigten Monocarbonsäuren mit 1 bis 12 Kohlenstoffatomen in der Hauptkette, die maximal drei Substituenten, ausgewählt aus den $C_1$-$C_4$-Alkylgruppen und den Chlor- oder Bromatomen, ausgewählt wird.

**11.** Verfahren nach Anspruch 10, dadurch gekennzeichnet, daß die Brönsted-Säure die Capronsäure ist.

**12.** Verfahren nach einem der vorangehenden Ansprüche, dadurch gekennzeichnet, daß das Mangan in Form einer Mangan(II)- oder einer Mangan(III)-Verbindung in das Reaktionsmedium eingebracht wird.

**13.** Verfahren nach Anspruch 12, dadurch gekennzeichnet, daß das Mangan in Form eines Acetats in das Reaktionsmedium eingebracht wird.

**14.** Verfahren nach Anspruch 12, dadurch gekennzeichnet, daß das Mangan in Form des Mangan(III)-acetylacetonats in das Reaktionsmedium eingebracht wird.

**15.** Verfahren nach einem der vorangehenden Ansprüche, dadurch gekennzeichnet, daß die Zersetzungstemperatur unter 120 °C liegt.

**16.** Verfahren nach einem der vorangehenden Ansprüche, dadurch gekennzeichnet, daß die Anfangskonzentration des Cyclohexylhydroperoxids 0,5 bis 8 Gew.-% beträgt.